# EUROPÄISCHE PATENTSCHRIFT

(11) **EP 1 709 881 B1**
(45) Veröffentlichungstag und Bekanntmachung des Hinweises auf die Patenterteilung: **04.03.2009**
(21) Anmeldenummer: 06007292.3
(22) Anmeldetag: 06.04.2006
(51) Int. Cl.: A43B 17/02, A43B 7/22, A61F 5/14, A43B 13/12

(54) **Fussbett**
Footbed
Semelle de renfort

(30) Priorität: 07.04.2005 DE 102005015863
(43) Veröffentlichungstag der Anmeldung: 11.10.2006
(73) Patentinhaber: Stumpf, Jürgen, 36037 Fulda (DE)
(72) Erfinder: Stumpf, Jürgen, 36037 Fulda (DE)
(74) Vertreter: Hebing, Norbert

(56) Entgegenhaltungen:
- EP-A- 0 060 353
- WO-A-03/090573
- WO-A-20/04086894
- DE-C- 463 383
- DE-U1- 8 700 681
- US-B1- 6 502 330

## Beschreibung

Die Erfindung bezieht sich auf ein Fußbett mit einer Versteifungseinlage, die sich vom Fersenbereich bis zum Zehenballen erstreckt und die im Rückfußbereich zwei nach hinten führende Korrekturzungen sowie auf der Seite der großen Zehe eine kurze Auflagezunge, die bis unterhalb zum Ballen des großen Zehes reicht, und an der Fußaußenseite eine lange Auflagezunge aufweist.

Ein solches Fußbett ist zum Beispiel in der DE 102 38 409 A1 beschrieben. Generell dienen solche Einlagen dazu, den Fuß eines Fußgängers bzw. Läufers zu stützen und um je nach Ausbildung des Fußskeletts übermäßige Pronation bzw. Supination zu verhindern.

Des Weiteren ist bekannt, das Fußbett mit elastischen Einlagen zu versehen, um die Auswirkungen von Stoßbelastungen, die insbesondere während einer Sportausübung wie Laufen oder Ballspielen dem Mehrfachen einer statischen Belastung entsprechen können, zu verringern. Hierbei handelt es sich stets um passive, von außen auf den Fuß einwirkende Maßnahmen, die aber häufig außer Acht lassen, dass die Fußstabilisierung auch aktiv, d. h. durch die Muskulatur des Fußes und des Unterschenkels erfolgt. Diese aktive Fußstabilisierung hat darüber hinaus den Vorteil, dass sie dynamisch erfolgt, d. h. wesentlich deutlicher dem Bewegungsablauf beim Laufen, d. h. beim Abrollen des Fußes folgt. Entscheidend für eine solche Anpassung der Muskelspannung an den Bewegungsablauf ist es, dass auf den Körper bzw. auf die Fußsohle Reize ausgeübt werden, die signalisieren, welchen Belastungen der Fuß momentan ausgesetzt ist bzw. in welcher Phase des Bewegungsablaufes sich der Fuß befindet.

Dementsprechend kann die Fuß- und Beinmuskulatur die Fußstabilität so einstellen, dass ein effektives Laufen möglich ist.

In den Dokumenten DE 87 00 681 U1, EP 0 060 353 A, DE 463 383 C und WO 03/090573 sind jeweils Einlagen mit einer bereichsweisen Polsterung eines Grundkörpers beschrieben. Die Polsterungen dienen vor allem dazu, den Druck auf druckempfindliche Bereiche der Fußsohle zu minimieren.

Die Erfindung beruht somit auf dem Problem, ein Fußbett zu schaffen, das sowohl bei langsamen als auch bei schnellen Bewegungsabläufen den Fuß in jeder Bewegungsphase ausreichend stabilisiert und führt.

Zur Lösung des Problems sieht die Erfindung vor, dass das Fußbett ausschließlich in einem Teilbereich wenigstens zwei punktuelle bzw. kleinflächige Auflagen auf der Versteifungseinlage aufweist, die aus einem elastischen Material bestehen, das sich unter einer stoßartigen Belastung versteift, wobei sich eine Auflage zur Bildung eines Druckpunktes unterhalb des Großzehballens befindet und eine weitere Auflage zur Bildung eines weiteren Druckpunktes unterhalb des fersennahen Bereichs der Fußwölbung befindet, und beide Auflagen so eingestellt sind, dass insbesondere beim schnellen Abrollen des Fußes punktuelle Nervenreize in den korrespondierenden Abschnitt der Fußsohle ausgelöst werden, die zu einer Anspannung von Muskeln im Fuß als auch im Unterschenkel führen.

In der US PS 4,471,538 ist ein Fußbett beschrieben, über dessen gesamte Fläche Kissen mit reoplexen Fluiden zur Stoßdämpfung verteilt sind, wobei sich die Form der Kissen an die Fußsohle des Schuhträgers anpasst. Ein gezieltes Auslösen von Nervenreizen wird damit nicht erzielt.

Ein bevorzugtes Material für die Auflagen ist Silikon. Dieses Material hat die Eigenschaft, bei einer langsamen, also einer quasi-statischen Belastung elastisch nachzugeben, während es bei einer raschen, d. h. stoßartigen Belastung ein höheres Elastizitätsmodul annimmt, sich also verhärtet. Dadurch passt sich sein Verhalten der Schnelligkeit des Bewegungsablaufes an. Bei einer langsamen Gehbewegung, bei der der Fuß wenig Unterstützung braucht, ist das Material weich und gewährt einen angenehmen Tragekomfort. Bei raschen Bewegungen, also zum Beispiel beim sportlichen Laufen, versteift sich das Material bei jeder Belastung und erhöht die Führung für den Fuß.

Entscheidend ist aber ein weiterer Aspekt dieser Auflagen. Werden diese erfindungsgemäß gezielt punktuell in einem Teilbereich des Fußbettes eingebaut, können beim Laufen die jeweiligen Fußsohlenbereiche durch einen von der Auflage herrührenden Druck stimuliert werden, so dass Reize ausgelöst werden, die letztendlich zu einer Anspannung von bestimmten Muskeln im Fuß, aber auch im Unterschenkel führen, was letztendlich zu einer Stabilisierung des gesamten Fußskelettes führt.

Ein solcher Bereich ist zum Beispiel die Fläche unterhalb der Fußwölbung. Dieser Bereich wird aktiviert, nachdem der Fuß mit der Ferse aufgesetzt wurde und nun mit dem Abrollvorgang beginnt. Der erste Kontakt ist der Bereich dieser Fußwölbung, der der Ferse besonders nahe liegt. Hier bietet sich an, die sich ggf. unter der gesamten Fußwölbung befindende Auflage erhaben auszuführen, um eine punktuelle Auflage zu bilden, die als Druckpunkt dient und einen entsprechenden Reiz auslöst. Ein weiterer Druckpunkt, den man als Erinnerungspunkt bezeichnen könnte, ist der Bereich der Großzehballen. Hier wird in der Endphase des Abrollens ein verzögerter Reiz ausgelöst, der die oben angesprochene Muskulatur nochmals reizt und zur Beibehaltung der Spannung animiert, bzw. so einstellt, dass sich der Fuß für das Abstoßen des Fußes vom Boden in einer optimalen Spannung befindet.

Durch die spezielle Gestaltung des Materials, nämlich die Versteifung bei einer stoßartigen Belastung, wird die Reizwirkung abhängig von der Bewegungsgeschwindigkeit. Solange das Material bei einer langsamen Bewegung weich bleibt, ist die Reizwirkung gering, sie wird umso stärker, je schneller der Bewegungsablauf erfolgt, wobei gerade bei solchen Bewegungsabläufen eine hohe Reizwirkung notwendig ist.

Insbesondere bei einer flächigen Ausführung des Materials ergibt sich aber die oben erwähnte wechselwirkung zwischen elastischer Lagerung des Fußes und harter Abstützung. Der Bereich, der hierfür insbesondere in Frage kommt, ist der gesamte Bereich der Fußwölbung. Um zusätzlich die beschriebene punktuelle Reizwirkung am Großzehballen und am fersennahen Bereich der Fußsohle zu erhalten, wird dort die Auflage verdickt ausgeführt, um die Druckpunkte für die beschriebene Reizwirkung zu bilden.

Im Folgenden soll anhand eines Ausführungsbeispiels die Erfindung näher erläutert werden.

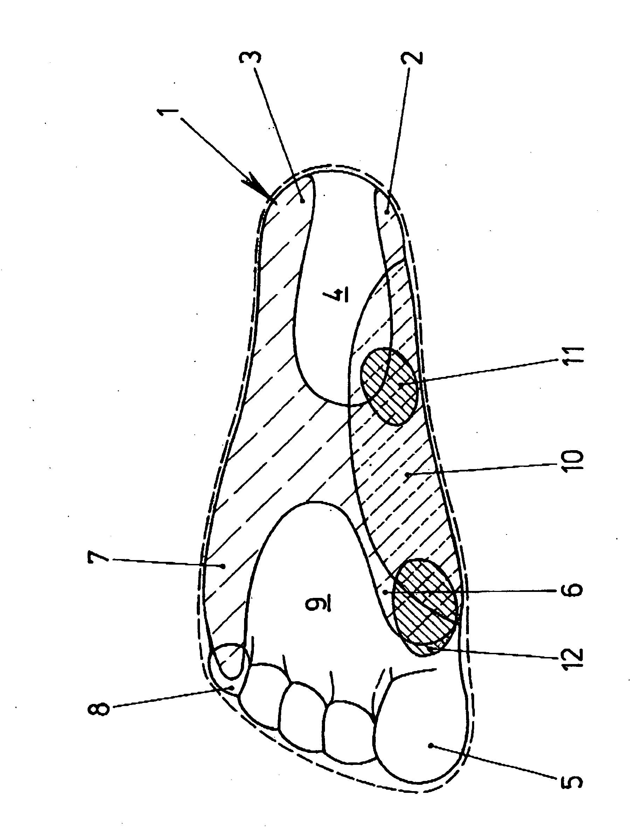

Die Zeichnung zeigt eine in die Kontur eines Fußes eingezeichnete, biegeelastische versteifungseinlage 1, die beispielsweise aus kohlefaserverstärktem Kunststoff besteht. Die Versteifungseinlage 1 hat im Rückfußbereich zwei nach hinten führende Korrekturzungen 2, 3, zwischen denen sich eine nach hinten hin offene Ausnehmung 4 befindet. An der Seite der großen Zehe 5 des Fußes hat die Versteifungseinlage 1 eine kurze Auflagezunge 6, die bis unterhalb des nicht gezeigten Ballens der großen Zehe 5 reicht. An der Fußaußenseite hat die Versteifungseinlage 1 eine lange Auflagezunge 7, die bis zum vorderen Ende der kleinen Zehe 8 reicht. Zwischen den Auflagezungen 6, 7 verläuft eine bis in den Mittelfußbereich führende Einbuchtung 9.

Auf dieser Einlage, also näher am Fuß, befindet sich im punktiert dargestellten Bereich eine Auflage 10, die aus Silikon besteht, welches die Eigenschaft hat, bei einer stoßartigen raschen Belastung sich steifer zu verhalten als bei einer langsamen, quasi statischen Belastung. Die Abstützung des Fußgewölbes variiert somit mit der Geschwindigkeit des Bewegungsablaufs.

Die Auflage 10 erfährt in zwei Bereichen jeweils eine Verdickung, nämlich im fersennahen Bereich der Fußwölbung und unter dem Großzehballen. Die erstgenannte Verdickung bildet einen ersten Druckpunkt, der als Reizpunkt 11 bezeichnet wird. Die zweitgenannte zweite Verdickung unterhalb des Großzehballens bildet einen als Erinnerungspunkt 12 bezeichneten Druckpunkt. Beim Abrollen des Fußes werden die entsprechenden Stellen der Fußsohle stimuliert und geben, wie Versuche gezeigt haben, entsprechende Reize an die Muskulatursteuerung weiter, die eine angepasste dynamische Vorspannung der Fußmuskulatur, aber auch der Unterschenkelmuskulatur bewirkt, was eine positive Auswirkung auf die Fußstabilität hat. Beim Abrollen des Fußes erfolgt somit eine erste Stimulation am Druckpunkt 11, eine Erinnerung und Verstärkung dieses Reizes im Fußballen am Erinnerungspunkt 12. Da die Auflagen aus Silikon bestehen und sich diese Bereiche in ihrer Steifigkeit geschwindigkeitsabhängig verhalten, wird die Reizwirkung bei schnellen Bewegungsabläufen verstärkt.

### Bezugszeichenliste

- 1: versteifungseinlage
- 2: Korrekturzunge
- 3: Korrekturzunge
- 4: Ausnehmung
- 5: große Zehe
- 6: kurze Auflagezunge
- 7: lange Auflagezunge
- 8: Zehe
- 9: Einbuchtung
- 10: Auflage
- 11: Reizpunkt
- 12: Erinnerungspunkt

## Patentansprüche

1. Fußbett mit einer Versteifungseinlage (1), die sich vom Fersenbereich bis zu den Zehenballen erstreckt und die im Rückfußbereich zwei nach hinten führende Korrekturzungen (2,3) sowie auf der Seite der großen Zehe eine kurze Auflagezunge (6), die bis unterhalb zum Ballen des großen Zehes reicht, und an der Fußaußenseite eine lange Auflagezunge (7) aufweist, **dadurch gekennzeichnet, dass** das Fußbett ausschließlich in einem Teilbereich wenigstens zwei punktuelle bzw. kleinflächige Auflagen (11, 12) auf der Versteifungseinlage aufweist, die aus einem elastischen Material bestehen, das sich bei einer stoßartigen Belastung versteift, wobei sich eine Auflage (12) zur Bildung eines Druckpunktes unterhalb des Großzehballens befindet und eine weitere Auflage (11) zur Bildung eines weiteren Druckpunktes unterhalb des fersennahen Bereichs der Fußwölbung befindet, und beide Auflagen (11, 12) so eingestellt sind, dass insbesondere beim schnellen Abrollen des Fußes punktuelle Nervenreize in den korrespondierenden Abschnitt der Fußsohle ausgelöst werden, die zu einer Anspannung von Muskeln im Fuß als auch im Unterschenkel führen.

2. Fußbett nach Anspruch 1, **dadurch gekennzeichnet, dass** das Material der Auflagen (11,12) Silikon ist.

3. Fußbett nach einem der vorhergehenden Ansprüche, **dadurch gekennzeichnet, dass** sich eine dritte Auflage (10) unterhalb der Fußwölbung befindet und eine der Fußwölbung entsprechende Ausdehnung besitzt, wobei diese Auflage (10) am Großzehballen und am fersennahen Bereich der Fußsohle verdickt ausgeführt ist, um die punktuellen bzw. Kleinflächigen Auflagen (11,12) zu bilden, durch die entsprechende Druckpunkte entstehen.

## Claims

1. Footbed with a reinforcement insert (1) which extends from the heel region up to the balls of the toes and which has two correction tongues (2, 3) leading toward the rear in the rear foot region and a short support tongue (6) on the side of the big toes, which reaches to beneath the ball of the big toe, and a long support tongue (7) on the outer side of the foot, **characterized in that** the footbed has exclusively in a partial region at least two point- or small area supports (11, 12) on the reinforcement insert, which consist of an elastic material which stiffens on a percussive loading, with one support (12) being situated to form a pressure point beneath the ball of the big toe and a further support (11) being situated to form a further pressure point beneath the region of the foot arch close to the heel, and both supports (11, 12) being adapted such that, especially during rapid rolling of the foot, point nerve stimuli are activated in the corresponding section of the foot sole, which lead to a tensing of muscles in the foot and also in the lower leg.

2. Footbed according to Claim 1, **characterized in that** the material of the supports (11, 12) is silicone.

3. Footbed according to any of the preceding claims, **characterized in that** a third support (10) is situated beneath the foot arch and has an extent corresponding to the foot arch, this support (10) being formed so as to be thickened at the ball of the big toe and at the region of the foot sole close to the heel, in order to form the point- or small area supports (11, 12) through which corresponding pressure points are produced.

## Revendications

1. Semelle plantaire avec un insert de renfort (1) qui s'étend de la zone du talon vers la pulpe des orteils et qui dans la zone de l'arrière-pied comporte deux languettes de correction (2, 3) menant vers l'arrière, ainsi que sur le côté du gros orteil comporte une courte languette d'appui (6), qui arrive jusqu'en dessous de la pulpe du gros orteil et sur le côté extérieur du pied comporte une longue languette d'appui (7), **caractérisée en ce que** la semelle plantaire comporte exclusivement dans une zone partielle au moins deux appuis (11, 12) ponctuels ou de faible surface sur l'insert de renfort, qui consistent dans une matière élastique, qui se rigidifie lors d'une contrainte par à-coups, un appui (12) se trouvant sous le gros orteil, pour former un point de pression sous la pulpe du gros orteil et un autre appui (11) se trouvant sous la zone proche du talon de la cambrure du pied, pour former un autre point de pression, et les deux appuis (11, 12) étant réglés de sorte que notamment lors d'un roulement rapide du pied, des stimuli nerveux ponctuels qui mènent à une tension de muscles dans le pied, tout comme dans la jambe soient déclenchés dans la section correspondante de la semelle.

2. Semelle plantaire selon la revendication 1, **caractérisée en ce que** la matière des appuis (11, 12) est du silicone.

3. Semelle plantaire selon l'une quelconque des revendications précédentes, **caractérisée en ce qu'**un troisième appui (10) se trouve sous la cambrure du pied et présente une extension correspondant à la cambrure du pied, ledit appui (10) étant réalisé en version surépaissie à la pulpe du gros orteil et dans la zone proche du talon de la semelle, pour former les appuis ponctuels ou à faible surface (11, 12) qui donnent naissance aux points de pression correspondants.
